# EUROPEAN PATENT SPECIFICATION

(11) **EP 2 032 720 B1**
(45) Date of publication and mention of the grant of the patent: **21.05.2014**
(21) Application number: 07747744.6
(22) Date of filing: 01.06.2007
(51) Int. Cl.: C12Q 1/68

(54) **FAST ASSIGNMENT OF ADEQUATE NEOADJUVANT CHEMOTHERAPY FOR BREAST CANCER PATIENTS BASED ON THE IDENTIFICATION OF CONSTITUTIONAL BRCA1 MUTATIONS**
SCHNELLE ZUWEISUNG EINER ANGEMESSENEN NEOADJUVANS-CHEMOTHERAPIE FÜR BRUSTKREBSPATIENTEN AUF DER GRUNDLAGE DER IDENTIFIZIERUNG KONSTITUTIONELLER BRCA1-MUTATIONEN
CHOIX RAPIDE D'UNE CHIMIOTHÉRAPIE PRÉOPÉRATOIRE ADÉQUATE POUR DES PATIENTES AYANT UN CANCER DU SEIN À PARTIR DE L'IDENTIFICATION DE MUTATIONS CONSTITUTIONNELLES DE BRCA1

(30) Priority: 01.06.2006 PL 37982706
(43) Date of publication of application: 11.03.2009
(73) Proprietor: Pomorski Uniwersytet Medyczny, 70-204 Szczecin (PL); Byrski, Tomasz, 70-115 Szczecin (PL); Gronwald, Jacek, 70-115 Szczecin (PL); Lubinski, Jan, PL-71-253 Szczecin (PL); Huzarski, Tomasz, 70-115 Szczecin (PL); Narod, Steven, Toronto, Ontario M5G 1N8 (CA)
(72) Inventor: BYRSKI, Tomasz, PL-70-115 Szczecin (PL); GRONWALD, Jacek, PL-70-115 Szczecin (PL); LUBINSKI, Jan, PL-71-253 Szczecin (PL); HUZARSKI, Tomasz, PL-70-115 Szczecin (PL); NAROD, Steven, Ontario M5G 1N8 (CA)
(74) Representative: Witek, Rafal
(86) International application number: PCT/PL2007/000035
(87) International publication number: WO 2007/139411

(56) References cited:
- WO-A1-2004/042080
- FRIEDMAN L S ET AL: "CONFIRMATION OF BRCA1 BY ANALYSIS OF GERMLINE MUTATIONS LINKED TO BREAST AND OVARIAN CANCER IN TEN FAMILIES" NATURE GENETICS, NEW YORK, NY, US, vol. 8, no. 12, December 1994 (1994-12), pages 399-404, XP000616433 ISSN: 1061-4036
- TASSONE P ET AL: "BRCA1 expression modulates chemosensitivity of BRCA1-defective HCC1937 human breast cancer cells" BRITISH JOURNAL OF CANCER, LONDON, GB, vol. 88, no. 8, 22 April 2003 (2003-04-22), pages 1285-1291, XP002448473 ISSN: 0007-0920
- CHABALIER C ET AL: "BRCA1 downregulation leads to premature inactivation of spindle checkpoint and confers paclitaxel resistance." CELL CYCLE (GEORGETOWN, TEX.) MAY 2006, vol. 5, no. 9, May 2006 (2006-05), pages 1001-1007, XP002477538 ISSN: 1551-4005
- KENNEDY RICHARD D ET AL: "The role of BRCA1 in the cellular response to chemotherapy" JOURNAL OF THE NATIONAL CANCER INSTITUTE (CARY), vol. 96, no. 22, 17 November 2004 (2004-11-17), pages 1659-1668, XP002477539 ISSN: 0027-8874
- THANGARAJU MUTHUSAMY ET AL: "BRCA1 facilitates stress-induced apoptosis in breast and ovarian cancer cell lines" JOURNAL OF BIOLOGICAL CHEMISTRY, vol. 275, no. 43, 27 October 2000 (2000-10-27), pages 33487-33496, XP002477540 ISSN: 0021-9258
- GORSKI B ET AL: "Breast cancer predisposing alleles in Poland" BREAST CANCER RESEARCH AND TREATMENT, vol. 92, no. 1, July 2005 (2005-07), pages 19-24, XP002477541 ISSN: 0167-6806
- BYRSKI T ET AL: "Response to neo-adjuvant chemotherapy in women with BRCA1-positive breast cancers" BREAST CANCER RESEARCH AND TREATMENT, vol. 108, no. 2, March 2008 (2008-03), pages 289-296, XP002477542 ISSN: 0167-6806

## Description

### FIELD OF THE INVENTION

Mode for optimizing the efficiency of breast cancer neoadjuvant chemotherapy, depending on the particular constitutional genotype characteristics of the gene BRCA1 in each patient. Generally, the invention concerns a new method to improve neoadjuvant therapy depending on a particular constitutional genotype. Subject of invention allow to synthesize DNA and identification of germline BRCA1 genetic abnormalities which are correlated with a significantly decreased clinical response to neoadjuvant chemotherapy based on taxane-derived cytostatics in breast cancer patients.

### BACKGROUND OF THE INVENTION

Constitutional mutations in the gene BRCA1 are the main factor responsible for high risk monogenic predisposition to breast and ovarian cancer (Ford et al. Am J Human Genet 1998; 62:676-89; Narod et al. Am J Hum Genet 1995; 56;254-64; Narod et al. Am J Hum Genet 1995; 57:957-8). Biological effects of BRCA1 belong to a reduced group of phenomena, where distinct abnormalities of just one protein lead to very critical consequences, almost independently of modifiers and environmental factors as evidenced here by the very high risk of breast and ovarian cancer risk of for BRCA1 mutation carriers throughout different human populations. To date hundreds of constitutional mutations have been described for BRCA1 (BIC database). Some of these are recurrent mutations with founder effect, i.e. show a population-specific profile. For example, and without loss of generality, there are founder mutations characteristic for the Ashkenazi Jewish (Tonin et al. Nat Medicine 1996; 2:1179-83), Finnish (Huusko et al. Am J Hum Genet 1998; 62:1544-8), Danish (Bergthorsson et al. J Med Genet 2001; 38:361-8), Italian (Russo et al. Breast Cancer Res Treat 2007; in press), English (Anglian Breast Cancer Study Group Br J Cancer 2000; :1301-8), Portuguese (Pexoto et al. Fam Cancer 2006; 5:379-87), Indian (Hedau et al. Breast Cancer Res Treat 2004; 88:177-86; Saxena et al. BMC Med Genet 2006; 7:75; Valarmathi et al. Hum Mutat 2004; 23:205), Japanese (Ikeda et al. Int J Cancer 2001; 91:83-8), Turkish (Yazici et al. Br J Cancer 2000; 83:737-42), Pakistani (Rashid et al. Int J Cancer 2006; 119:2832-9), Korean (Seo et al. Hum Mutat 2004; 24:350; Han et al. Clin Genet 2006; 70:496-501), Dutch (Peelen et al. Am J Hum Genet 1997; 60:1041-9; Petrij-Bosch et al. Nat Genet 1997; 17:341-5) or Canadians of French (Tonin et al. Am J Hum Genet 1998; 63:1341-51) and of English origin (Risch et al. Am J Hum Genet 2001; 68:700-10) among many others.

Analogous founder mutations can also be found in Slavic populations, as shared by e.g. the Polish (Gorski et al. Am J Hum Genet 2000; 66:1963-8), the Chech (Machackova et al. Cas Lek Cesk 2000; 139:635-7), the Latvian (Csokay et al. Hum Mutat 1999; 14:92), the Belarusian (Oszurek et al. Clin Genet 2001; 60:470-1), or the Russian (Tereschenko et al. Hum Mutat 2002; 19:184). The Polish patent application nr P. 335 917 describes founder mutations of the gene BRCA1 characteristic for the Slavic population. Another Polish patent application with nr P. 364 413 shows that ∼ 90% of all BRCA1 mutations in Poland belong to one out of three common mutations: BRCA1 ex.20 5382 ins C, BRCA1 ex.5 300T→G and BRCA1 ex.11 4153 del A.

[n summary, we can conclude that the current state of the art shows a strong correlation between germline mutations in the gene BRCA1 and predisposition to breast and ovarian cancer, whereas the influence of each particular mutation is different in different ethnic groups. Subject of this invention is a method for predicting response to neoadjuvant chemotherapy in breast cancer patients, who have already developed a tumor, dependent on their constitutional BRCA1 genotype.

Taxoids are diterpen compounds used in pharmacy mainly as cytostatics, e.g. paclitaxel (taxol) and docetaxel (taxotere). Paclitaxel is a highly complex molecule with several chiral bindings of carbon atoms. Paclitaxel was identified in 1960 in a research program of the National Insitute of Health (NIH) of the USA committed to the identification of active compounds out of 35 000 plant species. An extract from the bark of the Pacific Yew Tree, *Taxus brevifolia,* showed interesting cytostatic properties. In 1969 the active compound paclitaxel was isolated from the bark extract and in 1971 its chemical structure could be determined (Rowinsky et al. J National Can Inst 82:11247,1990).

In contrast to other substances as colchicine or vinca alkaloids, whose antitumoral properties rely on their ability to depolymerize the cell microtubules, paclitaxel mode of action prevents the depolymerization of the microtubules. In this way microtubules are stabilized to the extent that cell division is disrupted (Schiff et al. Nature 277:665,1979). The FDA allowed commercialization of paclitaxel in 1993 for chemotherapy in breast, ovarian, lung and prostate cancer, melanoma and leukaemia. The effectivity against ovarian, breast and lung cancer is around 30%, 50% and 20% respectively (David et al. J Nat Prod 53,1990).

Docetaxel also induces the assembly of microtubules, thus building a stable configuration during mitosis that prevents cell division (Katzung's Pharmacology, 9th Edition, 2004). Docetaxel was presented as a new-generation cytostatic, particularly efficient for chemotherapy against breast cancer (Piccart, Anticancer Drugs 4:7-11,1995). Currently, many other taxanes and their derivatives are known, as well as the extraction methods and the application for therapy against cancer: among others WO94/14787, US6916942, US6750246, US6610860, US6476242, US6369244, US6353120, US6248908, US6017935, US5977386, US5902822, US5840929, US5773464, US5773629, US4814470, US4857653, US4876399, US4942184, US4960790, US5278324, US5283253, US5352806. Thus, both taxane-derived chemotherapeutic drugs and their application will be considered as conventional from now on.

The current use of taxanes for chemotherapy against cancer is associated with side-effects. Such include neutropenia, alopecia, debilitation, pains in articulations and muscle tissue, skin reactions, anemia, water retention and even damages of the liver and the heart. It is also evidenced that therapeutical efficiency of those cytostatics is highly variable in different cancer patients. As can be deduced from the foregoing state of the art, an objective problem is the lack of a method that could reliably classify patients in groups of responders and non-responders towards cytostatics before therapy onset, in order to administrate directly an alternative drug, e.g. a DNA-damaging drug, to those patients which are less likely to respond to cytostatic therapy, thus improving therapy success and reducing the impact of unwanted side-effects.

A first method to cope with this specific problem is subject of patents WO2004042080 and W02005121786. Basing on in vitro studies, it is suggested that breast cancer cell lines with low BRCA1 activity are not responsive to chemotherapy with taxanes, and make thus recommendable to choose a DNA damaging agent as a chemotherapeutic agent, instead. Under the possible ways to determine a reduced BRCA1 activity, it is suggested the analysis of mutations in the gene BRCA1 in breast tumor biopsy material.

However, for the use of that mehod in clinical practice, some problems arise. A major objection is the generalization of the in vitro model to a human subject. The development of the tumor is influenced by many factors, such as permeability to the tumor cells, interstitial hypertension, metabolic degradation, immune response or angiogenesis among others, that greatly diverge between in vivo and in vitro studies and most remarkably the context of metabolites taken to and from the tumor site by blood circulation, e.g. regulator molecules expressed elsewhere, is completely absent in vitro. This divergences often account for a lack of correlation of the effect of anticancer drugs in vivo and vitro (Williams et al. Cancer Res 2000; 60:6045-51; Poondru et al. Invest New Drugs 2002; 20:23-33, McCready et al. J Natl Cancer Inst 1989; 81: 682-7).

Whenever neoadjuvant therapy is advisable, there is an immediate urge from the clinical point of view in determining the most efficient chemotherapy for the patient, since any delay in the application of the correct therapy reduces its chances of success. In this scenario, a method is needed that allows a fast decision-making for the oncologist.

The subject of the present invention is a method for predicting response to antitumoral taxane chemotherapy of a breast cancer patient, depending on his constitutional BRCA1 genotype, characterized by analysis of any genetic material obtained from the patient. In fact, as the method is focused on germline founder mutations, the prediction of the response to a possible future taxane therapy is already possible at a stage where the individual is just identified as predisposed to breast and ovarian cancer in the frame of a standard genetic scan for cancer associated markers, as is often performed e.g. in families with high cancer incidence or for family members of BRCA1 mutation carriers even in absence of family cancer aggregation.

As mutations of the BRCA1 gene it is understood mutations affecting the genetic sequence of the gene BRCA1, as well as flanking mutations in the direct neighbourhood of BRCA1, which are classified in the database of the Breast Cancer Information Core (BIC). The database is available in the internet under http://research.nhgri.nih.gov/bic/. The numeration system of the genetic sequence and the terminology to denominate the mutations used in the current patent comply with the established scientific terminology in this area. As founder mutations, it is understood those among the aforementioned ones, which appear with a characteristically high frequency in specific human populations with a common ethnical origin.

In the context of this invention, the patient is of known ethnical origin namely of Slavic origin, whereas the main founder mutations of BRCA1 gene observed in that population are 5382insC, 300T→G and 4153delA. As also disclosed, a patient may be Ashkenazi Jewish origin, wherein the main founder mutations of BRCA1 gene observed in that population are 185delAG and 5382insC. Exemplarily, a sample founder mutations characterizing different ethnic populations is summarized in table 1.

**Table 1. Sample of BRCA1 founder mutations**

| ***Founder BRCA1 mutations*** | ***Ethnic populations*** |
|---|---|
| S1503X | Pakistani |
| R1835X | Pakistani |
| 185insA | Pakistani |
| 185delAG | Ashkenazi Jewish, Hungarian, Indian, Hispanic, Pakistani |
| 5382insC | Ashkenazi Jewish, Turkish, Slavic, Hungarian |
| 300T→G | Slavic, Hungarian |
| 4153delA | Slavic |
| 3171ins5 (3166ins5) | Scandinavian |
| 2595delA | Scandinavian |
| 1806C→T | Scandinavian |
| 1201del11 | Scandinavian |
| 1135insA | Scandinavian |
| 1675delA | Scandinavian |
| 2804delAA | Dutch |
| Alu-mediated deletions ex13, ex22 | Dutch |
| 4446C→T | French Canadian |
| 2953del3+C | French Canadian |
| 3300delA | Thai |
| Asp67Glu | Thai |
| 2156delinsCC | Portuguese |
| 3450del4 | Portuguese |
| 2552delC | Hispanic |
| R1443X | Hispanic |
| S955X | Hispanic |
| IVS5→1G>A | Hispanic |
| Tyr978X | Non-Ashkenazi Jews |
| 943ins10 | West-African |
| IVS13+1G>A | West-African |

A genetic analysis of BRCA1 germline mutations based on population-specific panels of known founder mutations is particularly favorable, since it allows a highly reliable identification of the most common alterations in BRCA1 with conventional indirect techniques based on DNA or RNA within a question of hours. The mutations may be detected directly or indirectly at DNA, RNA or protein level, but particularly favorable in the context of this invention is the analysis of DNA or RNA for the indirect identification of mutations with one of the following techniques: ASO PCR (allele specific - polymerase chain reaction), SSCP (single-strand conformation polymorphism), ASA (allele specific analysis), RFLP-PCR (restriction fragment length polymorphism - polymerase chain reaction), Taqman RT-PCR (real-time PCR) or microarray technology. Examples of primers that can be used for the amplification of such sequences of the gene BRCA1 in the context of the present invention are presented in tables 2 and 3.

Analogously, it is also favorable the genetic analysis of BRCA1 germline mutations based on a larger, unspecific panel comprising all known BRCA1 founder mutations, or a sample of the most frequent ones, to be applied for patients with unknown ethnic origin.

In the context of this invention, the biological material subject of genetic analysis is not necessarily a tumor biopsy. In the contrary, somatic changes are more difficult to identify and mostly require time-consuming direct DNA or RNA sequencing techniques since, unlike founder mutations, they may occur at any position of the sequence. It is of critical relevance in clinical practice to assign the correct neoadjuvant chemotherapy, whenever needed, as soon as possible. Thus, the identification of the constitutional BRCA1 genotype should be preferably performed on biological material as easily available as possible, such is peripheral blood or saliva. This is a clear advantage in comparison with tumor biopsies, where the access to tumor material is more difficult and sometimes impossible. Moreover it constrains the spectrum of potential therapies already from the outset, even before development of a tumor, just basing on the constitutional genetic profile of the patient.

[n the context of this invention, taxane cytostatic drugs comprise paclitaxel, docetaxel and their known derivates and analogues, treated tumors comprise malignancies occurring with increased probability among BRCA1 mutation carriers such as prostate cancer, leukaemia, lymphoma and particularly breast and ovarian cancer, and mutations of the gene BRCA1 are constitutional mutations.

The invention is described in the following example of application, to better illustrate its relevance. However, the invention cannot be reduced to the mentioned examples.

### EXAMPLE

Reduced response to neoadjuvant taxane therapy in breast cancer patients, which curry a constitutional mutation in the gene BRCA1.

One of the key clinical issues that must be addressed in the treatment of hereditary breast cancer is choice of chemotherapy. Unlike chemotherapy given after surgery, the effects of neoadjuvant chemotherapy can be assessed quickly by measuring tumor size and lymph node status before and after treatment. Although response does not invariably predict a patient's ultimate outcome, rates of complete response correlate well with survival rates (Fisher et al. J Clin Oncol 1998; 16:2672- 85).

3479 unselected incident cases of invasive breast cancer were identified at 18 different hospitals in Poland during the study period. The medical records and pathology reports were reviewed locally at the study centre in Szczecin. Information was recorded on age at diagnosis, stage, grade and lymph-node status, estrogen-receptor status, multi-centricity and bilaterality. Pathology review of tumor blocks and/or paraffin-embedded slides were requested from the corresponding pathology centres. One or more specimens were obtained from 3136 of the 3472 patients. A central pathology review was conducted in Szczecin by two pathologists associated with the study. Pathologists were blinded to mutation status. Each case was reviewed with regard to histology (medullary, ductal, lobular, tubulolobular or other). Representative slides were obtained from 66 patients (78%). These were stained for estrogen-receptor, progesterone receptor and ERBB2. Where slides were unavailable, information on estrogen-receptor and progesterone receptor was abstracted from the pathology report.

A mutation analysis of the BRCA1 gene was carried out for mutations 4153delA and 5328insC by a multiplex allele-specific polymerase chain reaction (PCR) assay. A third common mutation (C61G) was detected with the help of a restriction enzyme site in exon 5 specific for that mutation. 3472 of the 3479 patients (99.8%) could be successfully genotyped.

Identification of several mutations may be carried out grouped or independently. In the former case the primers set comprises an oligonucleotide pair for the identification of the mutation and a second oligonucleotide pair for control (table 2). However, it is particularly favourable the use of primer sets for a single multiplex PCR reaction (table 3).

**Table 2. Primer sets for analysis of BRCA1 mutations with conventional PCR.**

| **Primer pairs** | **Primer ID** | **Function** | **Primer for sense strand [F] 5'->3'** | **Primer for antisense strand [R] 5'->3'** |
|---|---|---|---|---|
| Pair 1 for BRCA1 ex.20 5382 ins C | B1-5382INSCI1 | identification | CAC TTC CAT TGA AGG AAG CTT C | TAC CTT TCT GTC CTG GGG AT |
| Pair 2 for BRCA1 ex.20 5382 ins C | B1-5382INSCI2 | identification | TGA CGT GTC TGC TCC ACT TC | ACC TTT CTG TCC TGG GGA TT |
| Pair 3 for BRCA1 ex.20 5382 ins C | B1-5382INSCK1 | control | CAC TTC CAT TGA AGG AAG CTT C | CAA AGG GGA GTG GAA TAC AG |
| Pair 4 for BRCA1 ex.20 5382 ins C | B1-5382INSCK2 | | ATA TGA CGT GTC TGC TCC AC | CAA AGG GGA GTG GAA TAC AG |
| Pair 1 for BRCA1 ex.5 300T→G | B1EX5IK1 | identification/ control | CTC TTA AGG GCA GTT GTG AG | TTC CTA CTG TGG TTG CTT CC |
| Pair 2 for BRCA1 ex.5 300T→G | B1EXSIK2 | identification/ control | ATG GCT CTT AAG GGC AGT TG | TGT GGT TGC TTC CAA CCT AG |
| Pair 1 for BRCA1 ex.11 4153 delA | B1_4154DELAI 1 | identification | CAA AGG CAT CTC AGG AAC ATC | CAA GCC CGT TCC TCT TTC TCA |
| Pair 2 for BRCA1 ex.11 4153 delA | B1_4154DELAI 2 | identification | TTG GCT CAG GGT TAC CGA AG | AAG CCC GTT CCT CTT TGT CA |
| Pair 3 for BRCA1 sex. 11 4154 delA | B1_4154DELAK | control1 | TTG GCT CAG GGT TAC CGA AG | GTG CTC CCC AAA AGC ATA AAC |
| Pair 4 for BRCA1 ex.11 4153 delA | B1_4154DELAK | control2 | TCC TAG CCC TTT CCA TAC A | CACGTG CTC CCC AAA AGC ATA AAC |

**Table 3. Primer sets for analysis of BRCA1 mutations with multiplex PCR.**

| | |
|---|---|
| | Primer sets |
| 1 | B1EX5IK1F, B1EXSIK1R, B1_4154DELAI2F, B1_4154DELAI1R, B1-5382INSCI1F, B1-5382INSCI1R |
| 2 | B1EXSIK2F, B1EX5IK2R, B1_4154DELAK2F, B1_4154DELAI2R, B1-5382INSCK2F, B1-5382INSCI2R |
| 3 | B1EXSIK1F, B1EX5IK2R, B1_4154DELAI2F, B1_4154DELAI2R, B1-5382INSCI2F, B1-5382INSCI1R |

In order to achieve comparable amounts of amplified PCR products it is in some cases convenient to optimize the applied proportions of primers. Such optimization depends on several factors, e.g. type and activity of polymerase used or the length and composition of the amplified oligonucleotides, and can be achieved based on publicly available laboratory knowledge. Other components for the diagnostic set, besides the primers, include nucleotides, termostable polymerase and buffer for the polymerase reaction, that are necessary elements in the mixture of substances for the PCR reaction.

DNA is isolated from peripheral blood leucocytes by conventional methods, and then used as the matrix for the PCR reaction. Conventional diagnostic tests for mutations in the BRCA1 gene, adjusted for the Polish population, are based on multiplex ASO-PCR (mutations 4153delta and 5382insC) and RFLP (mutation C61G) methods.

The reaction mixture recommended for the aforementioned diagnostic test includes a mixture of primers responsible for
1. amplification of a fragment of exon 5 enclosing the location of the eventual mutation C61G. Additional PCR products are indicators for the quality of the PCR reaction and serve as internal controls. Restriction enzyme AvaII cuts the PCR product of exon 5 into two smaller fragments, whenever mutation C61G is present,
2. amplification of a fragment of exon 11 only in case mutation 4153delta is present in the analyzed material,
3. amplification of a fragment of exon 20 only in case mutation 5382insC is present in the analyzed material,
where the lengths of the PCR products for exons 5, 11 and 20 are chosen to allow for simple and unequivocal identification using electrophoresis in agarose gel.

Here, the reaction ASO-PCR was carried out in an automatic thermocycler (DNA ThermalCycler 9600 - Perkin Elmer). The mixture of substances for 25 µl volumen comprised: 1 µl (50ng-200ng) genomic DNA, 2.5 µl reaction buffer (100mM Tris-HCl, 500mM KCL, 15mM MgCl₂, 1mg/ml gelatin; pH 8.6), 2-14 pM of each primer, 200 µM of each desoxynucleotide (dATP, dCTP, dGTP and dTTP) and 1 U Taq DNA polimerase. For each reaction there are additionally 3 positive controls (control DNA from carriers of the mutations 5382insC, C16G and 4153delA) and 2 negative controls (control DNA from non-carriers and a control with no DNA at all).

Amplification takes place under the following conditions:
a) DNA denaturation at 95°C during 5 minutes,
b) 10 cycles consisting each of
   denaturation at 94°C during 30 seconds
   primer binding at 68-58°C during 30 seconds*
   elongation of complemetary DNA at 72°C during 35 seconds
c) 30 cycles consisting each of
   denaturation at 94°C during 30 seconds
   primer binding at 57°C during 30 seconds
   elongation of complemetary DNA at 72°C during 30 seconds
* - for the first 10 cycles the temperature for primer binding is decreased in 1.2°C for each following cycle (in the first cycle it took 68°C, in the second 66.8°C, in the third 65.6°C, in the fourth 64.4°C, in the fifth 63.2°C, in the sixth 62°C, in the seventh 60.8°C, in the eigth 59.6°C, in the nineth 58.4°C and in the tenth 57.2°C).

5µl of PCR reaction products were mixed with 10µl Stop buffer (Solution of saccharose stained with bromophenol blue) and subjected to electrophoresis in agarose gel (1.5% agarose SeaKem FMC, 1x bufor TBE, 25 µg/ml ethidium bromide) under 6V/cm for 30 min. The separated products in the gel were visualized with UV illumination.

820 women received neo-adjuvant chemotherapy. Among them, 44 were carriers of one of the above mentioned BRCA1 mutations.

Specific attention was paid to the size of the tumor, prior to and after neoadjuvant chemotherapy and the lymph node status. Pre-treatment tumor size was determined in all patients by a combination of clinical examination and mammography (for some patients ultrasound examinations were also performed). Post-treatment size was determined by pathology report.

Lymph node status was evaluated prior to and after treatment. Pre-treatment lymph node status was evaluated with a combination of clinical exam, ultrasound and fine needle aspiration. After treatment all patients underwent axillary dissection and node status was evaluated by pathology report.

Each study subject was classified, according to response, into complete response (no evidence of tumor after treatment, either locally or within the axillary nodes), partial response (residual tumor of size smaller than the original tumor) and no response (tumor size following treatment equal to, or larger than original tumor size). Patients who experienced a complete response had no residual tumor in the breast tissue upon pathology examination (pathologic complete response).

For each of the 44 BRCA1-positive cases who received neoadjuvant chemotherapy, a matched mutation-negative breast cancer control was selected. The non-carrier control also received neoadjuvant chemotherapy. Carriers and non-carriers were matched on centre, age at diagnosis (within one year) and year of birth (within one year). However, clinical information could only be obtained for 41 of the 44 matched controls. The statistical significance of group differences was assessed using Fisher's Exact Test. Subgroups were defined, based on the results of the ER, PR and ERBB2 immuno-staining and by the category of chemotherapy received (i.e. Taxane containing versus others).

Carrier cases and non-carrier controls are compared in Table 4. Cases and controls were similar with respect to age, tumor size and nodal status. tumors in BRCA1 carriers were more likely to be estrogen-receptor negative, progesterone-negative, ERBB2-negative than tumors in non-carriers (p<0.01 for each). 4 medullary cancers were seen in the BRCA1-positive group, versus none in the non-carriers (p=0.11).

Overall, 35 of the 44 BRCA1 carriers achieved a complete or partial response (80%), compared to 39 of 41 non-carriers (95%; p=0.05) (tables 5 and 6).

A statistically significant difference in the proportions of non-responders in carriers and non-carriers was observed among users of taxane-based regimens. Only 6 of the 15 BRCA1 carriers under docetaxel therapy had a response (complete or partial), compared to 12 of 12 noncarriers (p=0.001). All 29 mutation carriers treated with another treatment regimen (treatments described in table 5) had partial or complete response, compared to 27 of the 29 non-carriers controls. Thus, the inferior response rate to neoadjuvant chemotherapy among BRCA1 carriers was restricted to the subgroup of women given docetaxel. All women who received docetaxel also received doxorubicin (the standard protocol was doxorubicin 50mg/m2 with docetaxel 75 mg/m2, administered on the same day, for 4 cycles, at 21-day intervals).

These results evidence a decreased response to taxanes for BRCA1 carriers around 13-fold less than the controls (Peto OR: 0.074; 95%CI: 0.015-0.361), which is statistically significant (p=0.001). In contrast, there is no satistically significant difference in the efficacy of chemotherapeuticals not based on taxanes (Peto OR: 7.66; 95%CI: 0.50-125).

BRCA1 carriers were more likely than non-carriers to be negative for estrogen-receptor, progesterone receptor and ERBB2 (table 4). However, the response to docetaxel appeared to be dependent on the BRCA1 status, but not on the receptor status. 27 BRCA1 mutation carriers with ER-negative tumors received treatments that did not contain docetaxel and all 27 achieved a complete or partial response. 12 BRCA1 carriers with ER-negative tumors received docetaxel and doxorubicin. Only 5 experienced a complete or partial response (p=0.0002 for the difference). 4 non-carriers with ER-negative tumors received docetaxel and all 4 responded.

**Table 4. Characteristics of breast cancer among BRCA1 mutation carriers and matched non-carriers**

| | BRCA1 mutation carriers | | | Non-carriers | | |
|---|---|---|---|---|---|---|
| | | N=44 | | | N=41 | |
| Average age | | 42.3 years | | | 42.0 years | |
| Age groups | | | | | | |
| 20-30 | | 3 | 6.8% | | 1 | 2.4% |
| 31-40 | | 10 | 22.7% | | 14 | 34.2% |
| 41-50 | | 31 | 70.5% | | 26 | 63.4% |
| Tumor histology | | | | | | |
| Ductal | 24 | 54.5% | | 21 | 51.2% | |
| Lobular | 1 | 2.3% | | 6 | 14.6% | |
| Medullary | | 4 | 9.1% | | 0 | 0 |
| Other | | 15 | 34.1 % | | 14 | 34.2% |
| ER-Status | | | | | | |
| Positive | | 1 | 2.3% | | 18 | 43.9% |
| Negative | | 40 | 91% | | 18 | 43.9% |
| Missing | | 3 | 6.8% | | 5 | 12.9% |
| PR-Status | | | | | | |
| Positive | | 2 | 4.5% | | 11 | 26.8% |
| Negative | | 38 | 90.9% | | 23 | 56.1% |
| Missing | | 4 | 6.8% | | 7 | 17.1% |
| ERBB2-Status | | | | | | |
| Positive | | 8 | 18.2% | | 11 | 26.8% |
| Negative | | 26 | 59.1 % | | 18 | 43.9% |
| Missing | | 10 | 22.7% | | 12 | 29.3% |
| Multicentricity | | | | | | |
| Unicentric | | 23 | 52.3% | | 21 | 51.2% |
| Multicentric | | 6 | 13.6% | | 9 | 21.9% |
| Missing | | 15 | 34.1% | | 11 | 26.9% |
| Tumor size (cm) | | | | | | |
| <1cm | | 0 | 0% | | 0 | 0% |
| 1-2cm | | 4 | 9.1% | | 4 | 9.8% |
| 2-5cm | | 30 | 68.2% | | 26 | 63.4% |
| >5cm | | 9 | 20.5% | | 11 | 26.8% |
| Missing | | 1 | 2.2% | | 0 | 0 |
| Lymph node status | | | | | | |
| Negative | | 12 | 27.3% | | 11 | 26.8% |
| Positive | | 32 | 72.3% | | 29 | 70.7% |
| Missing | | 0 | 0 | | 1 | 2.5% |
| Family history on breast and/or ovarian cancer | | | | | | |
| Negative | | 4 | 9.1% | | 9 | 22% |
| Positive | | 37 | 84.1% | | 17 | 41.5% |
| Missing | | 3 | 6.8% | | 15 | 36.5% |

Thus, we observed that women with a BRCA1 mutation who received the spindle poison docetaxel in combination with doxorubicin as neo-adjuvant chemotherapy for breast cancer were significantly less likely to respond to the treatment than women with no mutation. In contrast, BRCA1 carriers who were treated only with alternative DNA-damaging chemotherapies, responded in the same proportion as non-carriers. These observations are consistent with the theory that the expression of the wild-type BRCA protein is necessary for cancer cells to respond to spindle poisons such as docetaxel. BRCA1 may increase cell sensitivity to spindle poisons by signalling a pro-apoptotic pathway in response to spindle damage. In the absence of functional BRCA1, the mitotic spindle checkpoint is not activated and apoptosis is not induced. Supporting that hypothesis, two groups reported that sensitivity to paclitaxel was increased when BRCA1 protein was reconstituted into the tumor cell lines (Lafarge et al. Oncogene 2001; 20:6597-606; Zhoue et al. Oncogene 2003; 22:2396-404) and a third group rendered MCF7 cells insensitive to paclitaxel with a premature inactivation of the spindle checkpoint due to BRCA1 protein downregulation (Chabalier et al. Cell Cycle 2003; 5:1001-7). In contrast, after treatment with DNA-damaging drugs such as anthracyclines, methotrexate and doxorubicin, BRCA1 contributes to DNA repair. The absence of functional BRCA1 protein in BRCA1-mutation carriers should result in poor DNA repair ability, and therefore to an enhanced sensitivity to drugs in this class.

In summary, it was evidenced that breast cancer patients being carriers of BRCA1 germline mutation were significantly less receptive for neoadjuvant cytostatic therapy with taxane-derived drugs than non-carriers, while responsiveness towards DNA-damaging drugs seemed not to be affected by the presence of BRCA1 mutation.

**Table 5. Characteristics of BRCA1 mutation carrier breast cancer patients treated with neoadjuvant chemotherapy**

| 1 | 2 | 3 | 4 | 5 | 6 | 7 | Receptors | | | 8 | Response to chemotherapy | | | Vital status |
|---|---|---|---|---|---|---|---|---|---|---|---|---|---|---|
| | | | | | | | ER (IHC) | PgR (IHC) | HER2 (IHC) | | | | | |
| | | | | | | | | | | | CR | PR | BO | |
| 1 | 46 | 2003 | 5382insC | CMF | 10.0 | 1.5 | n.d. | n.d. | n.d. | +/- | | + | | D |
| 2 | 35 | 2002 | C61G | CMF | 4.5 | 3.0 | - | - | +++ | +/+ | | + | | A |
| 3 | 47 | 2003 | 5382insC | CMF | 4.0 | 2.0 | - | - | - | +/+ | | + | | A |
| 4 | 41 | 2000 | 5382insC | CMF | 18. | 5.0 | - | - | n.d. | +/- | | + | | A |
| 5 | 48 | 1998 | 5382insC | CMFP | 4.0 | 2.0 | - | - | - | +/+ | | + | | A |
| 6 | 47 | 1998 | 5382insC | CMF | 3.5 | 1.0 | - | - | ++ | +/- | | + | | A |
| 7 | 47 | 2001 | 5382insC | CMFP | 3.0 | 0.5 | - | - | - | +/- | | + | | A |
| 8 | 48 | 2002 | C61G | CMFP | 2.5 | 0.5 | - | - | - | +/+ | | + | | A |
| 9 | 35 | 2002 | C61G | AC | 6.0 | 2.0 | - | - | - | +/+ | | + | | A |
| 10 | 45 | 2001 | 5382insC | AC | 2.0 | 1.4 | - | - | - | +/- | | + | | A |
| 11 | 46 | 1999 | 5382insC | AC | 3.5 | 1.0 | - | n.d. | n.d. | -/- | | + | | A |
| 12 | 45 | 2001 | 5382insC | AC | 4.0 | 1.3 | - | - | - | +/+ | | + | | A |
| 13 | 44 | 2002 | C61G | AC | 3.4 | 1.0 | - | + | - | -/- | | + | | A |
| 14 | 30 | 2002 | 5382insC | AC | 2.5 | n.d. | - | - | ++ | +/- | + | | | A |
| 15 | 46 | 2002 | 5382insC | FAC | 2.5 | 1.0 | - | - | - | +/- | | + | | A |
| 16 | 40 | 2003 | 5382insC | FAC | 4.5 | 1.5 | - | - | n.d. | +/- | | + | | A |
| 17 | 43 | 2003 | 5382insC | FAC | 5.0 | 3.0 | - | - | - | -/- | | + | | D |
| 18 | 39 | 1996 | C61G | FAC | 4.5 | 2.2 | - | - | ++ | -/- | | + | | A |
| 19 | 38 | 2002 | 5382insC | FAC | 3.0 | 1.0 | - | - | - | -/- | | + | | A |
| 20 | 33 | 2003 | 5382insC | FAC | 2.5 | 1.5 | - | - | ++ | -/- | | + | | A |
| 21 | 44 | 2000 | 5382insC | FAC | 3.0 | 1.5 | - | - | n.d. | -/- | | + | | A |
| 22 | 41 | 1997 | 5382insC | FAC | 15.0 | 7.0 | n.d. | n.d. | n.d. | +/+ | | + | | A |
| 23 | 43 | 2002 | 5382insC | FAC | 3.5 | 2.5 | - | - | - | -/- | | + | | A |
| 24 | 50 | 2003 | C61G | FAC | 6.0 | 3.5 | - | - | ++ | -/- | | + | | A |
| 25 | 43 | 2004 | 5382insC | FAC | 7.0 | n.d. | - | - | - | +/- | + | | | A |
| 26 | 45 | 2004 | 5382insC, | FAC | 5.0 | n.d. | - | + | - | +/- | + | | | A |
| 27 | 39 | 2004 | 5382insC | FAC | 5.0 | n.d. | - | - | n.d. | +/- | + | | | A |
| 28 | 31 | 2004 | C61G | AT | 8.0 | 8.0 | n.d. | n.d. | n.d. | +/+ | | | + | D |
| 29 | 30 | 2001 | 5382insC | AT | 4.5 | 5.0 | - | + | n.d. | +/+ | | | + | A |
| 30 | 44 | 2002 | 5382insC | AT | 1.8 | 1.8 | - | - | n.d. | +/+ | | | + | A |
| 31 | 48 | 2002 | C61G | AT | 3.5 | 3.5 | - | - | - | +/+ | | | + | A |
| 32 | 46 | 2002 | 5382insc | AT | 3.5 | 3.5 | ++ | - | - | -/- | | | + | A |
| 33 | 43 | 2003 | 5382insc | AT | 4.0 | 4.0 | - | - | - | -/- | | | + | A |
| 34 | 43 | 2003 | 5382insC | AT | 4.5 | 4.5 | - | - | - | +/+ | | | + | A |
| 35 | 49 | 2003 | 4153delA | AT | 2.5 | 2.5 | - | - | - | +/+ | | | + | D |
| 36 | 43 | 2003 | C61G | AT | 6.5 | 13.0 | - | - | - | +/+ | | | + | D |
| 37 | 46 | 2002 | 5382insC | AT | n.d. | n.d. | - | - | +++ | +/+ | | + | | A |
| 38 | 48 | 2003 | 5382insC | AT | 3.4 | 1.7 | - | - | - | +/+ | | + | | A |
| 39 | 30 | 2001 | C61G | AT | 10.0 | 1.8 | - | - | - | -/- | | + | | A |
| 40 | 45 | 2001 | 4153delA | AT | 2.0 | 1.0 | n.d. | n.d. | n.d. | +/+ | | + | | A |
| 41 | 38 | 2002 | 5382insC | AT | 3.0 | 2.0 | - | - | - | +/+ | | + | | A |
| 42 | 45 | 2004 | 5382insC | AT | 2.5 | n.d. | - | - | +++ | +/+ | | + | | A |
| 43 | 49 | 2000 | 5382insC | CMF | 5.0 | 2.5 | - | - | n.d. | +/+ | | + | | A |
| 44 | 35 | 2001 | 5382insC | CMFP | 2.0 | n.d. | - | - | - | +/+ | | + | | A |

| | | | | | | | | | | | | | | |
|---|---|---|---|---|---|---|---|---|---|---|---|---|---|---|
| Abbreviations: CMF - cyclophosphamide (C), methotrexate (M) and fluorouracil (5-FU) 5CMFP - cyclophosphamide (C), methotrexate (M), fluorouracil (5-FU) and prednisone (P) AC - doxorubicin (Adriamycin, A) and cyclophosphamide (C) FAC - fluorouracil (5-FU), doxorubicin (Adriamycin, A) and cyclophosphamide (C) AT - doxorubicin (Adriamycin, A) and docetaxel (Taxotere, T) NA - vinorelbine (Navelbine, N) and doxorubicin (Adriamycin, A) A - alive D - deceased n.d. - no data | | | | | | | | | | | | | | |

**Table 6. Characteristics of non-carrier breast cancer patients treated with neoadjuvant chemotherapy**

| | | | | | | | | | | | | | | |
|---|---|---|---|---|---|---|---|---|---|---|---|---|---|---|
| Abbreviations: like in foregoing table. | | | | | | | | | | | | | | |

| 1 | 2 | 3 | 4 | 5 | 6 | 7 | Receptors | | | 8 | Response to chemotherapy | | | Vital status |
|---|---|---|---|---|---|---|---|---|---|---|---|---|---|---|
| | | | | | | | | | | | C R | PR | BO | |
| | | | | | | | ER (IHC) | PgR (IHC) | HER2 (IHC) | | | | | |
| 1 | 37 | 1997 | - | CMF | 5.0 | 2.0 | + | - | - | +/+ | | + | | D |
| 2 | 37 | 2003 | - | CMF | 6.0 | 1.0 | - | - | - | -/- | | + | | A |
| 3 | 32 | 2003 | - | CMF | 2.5 | 1.5 | - | - | - | +/+ | | + | | A |
| 4 | 47 | 2002 | - | CMF | 2.8 | 0.5 | n.d. | n.d. | n.d. | +/+ | | + | | A |
| 5 | 47 | 1998 | - | CMF | 3.0 | 1.5 | + | - | - | -/- | | + | | A |
| 6 | 50 | 2002 | - | CMF | 4.8 | 2.2 | - | - | ++ | +/+ | | + | | A |
| 7 | 49 | 2003 | - | CMFP | 2.8 | 1.8 | - | - | - | +/+ | | + | | A |
| 8 | 45 | 1998 | - | CMFP | 1.6 | 1.0 | + | - | - | +/+ | | + | | A |
| 9 | 45 | 2002 | - | AC | 4.0 | 1.0 | ++ | + | ++ | +/+ | | + | | D |
| 10 | 40 | 1997 | - | CMFP | 3.8 | n.d. | n.d. | n.d. | n.d. | +/+ | | + | | A |
| 11 | 48 | 2002 | - | AC | 3.0 | 1.5 | ++ | - | - | -/- | | + | | A |
| 12 | 46 | 1999 | - | AC | 4.5 | 1.0 | + | n.d. | n.d. | +/+ | | + | | D |
| 13 | 47 | 2003 | - | AC | 3.5 | 2.5 | ++ | - | - | -/- | | + | | A |
| 14 | 46 | 2002 | - | AC | 1.5 | 0.8 | - | - | n.d. | -/- | | + | | A |
| 15 | 36 | 2002 | - | AC | 2.0 | 2.0 | - | - | +++ | +/+ | | | + | A |
| 16 | 44 | 2002 | - | FAC | 7.0 | 4.0 | + | + | - | -/- | | + | | A |
| 17 | 44 | 2002 | - | FAC | 3.5 | 2.5 | + | ++ | - | +/+ | | + | | A |
| 18 | 45 | 2003 | - | FAC | 3.0 | 1.2 | - | - | +++ | +/+ | | + | | D |
| 19 | 48 | 2003 | - | FAC | 2.0 | 1.2 | - | - | - | +/+ | | + | | A |
| 20 | 40 | 2004 | - | FAC | 8.0 | 0.8 | - | - | n.d. | -/- | | + | | A |
| 21 | 40 | 2000 | - | FAC | 4.5 | 3.0 | ++ | + | n.d. | -/- | | + | | A |
| 22 | 33 | 2003 | - | FAC | 4.0 | n.d. | - | - | - | +/- | + | | | A |
| 23 | 45 | 2003 | - | FAC | 4.0 | 1.5 | ++ | + | - | +/n.d. | | + | | A |
| 24 | 43 | 2003 | - | FAC | 8.0 | 5.0 | - | - | +++ | +/+ | | + | | A |
| 25 | 46 | 2003 | - | FAC | 6.0 | n.d. | - | - | n.d. | -/- | + | | | A |
| 26 | 24 | 2003 | - | FAC | 5.0 | 10.0 | - | - | +++ | +/+ | | | + | A |
| 27 | 43 | 2003 | - | FAC | 7.0 | 5.0 | - | - | +++ | +/+ | | + | | D |
| 28 | 39 | 2003 | - | NA | 4.0 | 1.5 | + | ++ | - | +/+ | | + | | A |
| 29 | 48 | 2001 | - | NA | 3.5 | 1.5 | ++ | +++ | - | +/+ | | + | | A |
| 30 | 34 | 2002 | - | AT | 10.0 | 2.5 | ++ | ++ | - | -/- | | + | | D |
| 31 | 31 | 2002 | - | AT | 5.5 | 2.0 | ++ | + | +++ | +/+ | | + | | D |
| 32 | 32 | 2003 | - | AT | 3.5 | 2.0 | - | - | - | +/+ | | + | | A |
| 33 | 45 | 2001 | - | AT | 4.5 | 2.5 | - | - | n.d. | +/- | | + | | A |
| 34 | 47 | 2003 | - | AT | 6.0 | 3.5 | +++ | + | ++ | +/+ | | + | | A |
| 35 | 44 | 2001 | - | AT | 5.0 | 1.5 | - | n.d. | n.d. | +/+ | | + | | A |
| 36 | 43 | 2003 | - | AT | 6.5 | 4.5 | n.d. | n.d. | n.d. | n.d. | | + | | A |
| 37 | 31 | 2003 | - | AT | 2.5 | 0.7 | + | - | +++ | +/+ | | + | | A |
| 38 | 46 | 2002 | - | AT | 2.8 | 1.4 | n.d. | n.d. | n.d. | -/- | | + | | A |
| 39 | 38 | 2003 | - | AT | 5.0 | 2.0 | + | +++ | - | +/+ | | + | | A |
| 40 | 49 | 2000 | - | AT | 5.8 | 3.8 | - | - | +++ | +/+ | | + | | A |
| 41 | 48 | 2001 | - | AT | 4.5 | 2.0 | n.d. | n.d. | n.d. | +/- | | + | | A |

## Claims

1. A method for early detection of reduced clinical response towards neoadjuvant chemotherapy with taxane cytostatic drugs in breast cancer patients, wherein the breast cancer patients are from Slavic population, which comprises detecting BRCA1 gene founder mutations 5382insC, 300T→G and 4153delA in a biological sample from the analyzed patient, wherein the presence of a 5382insC, 300T→G or 4153delA carrier genotype is indicative of significantly decreased clinical response of patients to neoadjuvant chemotherapy with taxane cytostatic drugs.

2. A method according to claim 1, wherein Slavic population comprises Polish, Czech, Latvian, Belarusian and Russian patients.

3. The method of claim 1, wherein 5382insC, 300T→G and 4153delA mutations are identified by comparison of the structure of the altered BRCA1 variant with the wild type.

4. The method of claims 1, wherein detecting BRCA1 gene founder mutations 5382insC, 300T→G and 4153delta is based on analysis of DNA, RNA or proteins.

5. The method according to claim 4, wherein DNA or RNA testing is performed using any conventional technique of direct mutation detection selected among sequencing or indirect mutation detection, selected among ASA-, ASO-, RFLP-PCR, Taqman RT-PCR or microarrays methods based on common founder mutation panels.

6. The method according to claim 4, wherein the presence of the polypeptide encoded by the BRCA1 gene with germline alteration is detected with the use of antibodies.

7. The method of claim 1, wherein the taxane cytostatic drug is selected from a taxoid or paclitaxel or docetaxel.

## Patentansprüche

1. Ein Verfahren zur Früherkennung des verminderten therapeutischen Effektes der neoadjuvanten Chemotherapie mit Zytostatika aus der Gruppe der Taxane bei Brustkrebspatienten, die zu der slawischen Bevölkerung gehören, wobei das Verfahren die Detektion von Gründer-Mutationen 5382insC, 300T→G und 4153delA im BRCA1-Gen, in einer biologischen Probe des zu analysierenden Patienten umfasst, wobei das Vorhandensein des Genotyps mit 5382insC, 300T→G oder 4153delA Rückschlüsse auf einen signifikant verminderten therapeutischen Effekt der neoadjuvanten Chemotherapie mit Zytostatika aus der Gruppe der Taxane beim Patienten schließen lässt.

2. Verfahren nach Anspruch 1, wobei die slawische Bevölkerung polnische, tschechische, lettische, weißrussische und russische Patienten umfasst.

3. Verfahren nach Anspruch 1, wobei 5382insC, 300T→G und 4153delA Mutationen durch einen Strukturvergleich zwischen der veränderten BRCA1-Variante und dem Wildtyp identifiziert werden.

4. Verfahren nach Anspruch 1, wobei die Detektion der Gründer-Mutationen 5382insC, 300T→G und 4153delA auf DNA-, RNA- oder Proteinanalysen beruht.

5. Verfahren nach Anspruch 4, wobei die DNA- und RNA-Analysen durchgeführt werden, indem eine der herkömmlichen Technologien zum direkten Mutationsnachweis angewendet wird, ausgewählt aus Sequenzierung, oder, indem ein indirekter Mutationsnachweis, der aus ASA-, ASO-, RFLP-PCR oder Taqman RT-PCR ausgewählt wird, angewendet wird, oder indem Microarray-Technologien angewendet werden, die Panels mit den gängigen Gründer-Mutationen verwenden.

6. Verfahren nach Anspruch 4, wobei das Vorhandensein des Polypeptids, das vom BRCA1-Gen mit einer Keimbahn-Mutation kodiert wird, durch die Verwendung von Antikörpern detektiert wird.

7. Verfahren nach Anspruch 1, wobei ein Taxoid, Paclitaxel oder Docetaxel als Zytostatikum aus der Gruppe der Taxane ausgewählt wird.

## Revendications

1. Procédé de détection précoce d'une réduction de la réponse clinique à une chimiothérapie néoadjuvante avec des médicaments cytostatiques à taxanes chez des patients souffrant d'un cancer du sein, les patients souffrant d'un cancer du sein étant d'une population slave, qui comprend la détection des mutations fondatrices du gène BRCA1 5382insC, 300T→G et 4153delA dans un échantillon biologique provenant du patient analysé, dans lequel la présence d'un génotype de porteur de 5382insC, 300T→G ou 4153delA indique une diminution significative de la réponse clinique des patients à une chimiothérapie néoadjuvante avec des médicaments cytostatiques à taxanes.

2. Procédé selon la revendication 1, dans lequel la population slave comprend des patients polonais, tchèques, lettons, biélorusses et russes.

3. Procédé selon la revendication 1, dans lequel les mutations 5382insC, 300T→G et 4153delA sont identifiées par comparaison de la structure du variant modifié de BRCA1 avec le type sauvage.

4. Procédé selon la revendication 1, dans lequel la détection des mutations fondatrices du gène BRCA1 5382insC, 300T→G et 4153delA est basée sur l'analyse d'ADN, d'ARN ou de protéines.

5. Procédé selon la revendication 4, dans lequel l'analyse d'ADN ou d'ARN est effectuée en utilisant toute technique traditionnelle de détection directe de mutations choisie parmi le séquençage ou de détection indirecte de mutations, choisie parmi des procédés de PCR ASA, ASO, RFLP, RT-PCR Taqman ou des micropuces, basés sur des panels de mutations fondatrices courantes.

6. Procédé selon la revendication 4, dans lequel la présence du polypeptide codé par le gène BRCA1 avec modification de la lignée germinale est détectée avec l'utilisation d'anticorps.

7. Procédé selon la revendication 1, dans lequel le médicament cytostatique à base de taxane est choisi parmi un taxoïde ou le paclitaxel ou le docétaxel.
